# EUROPEAN PATENT APPLICATION

(11) **EP 4 529 852 A1**
(43) Date of publication of application: **02.04.2025**
(21) Application number: 23200919.1
(22) Date of filing: 29.09.2023
(51) Int. Cl.: A61B 6/04, A61B 5/055

(54) **SUBJECT SUPPORT WITH MISALIGNMENT COMPENSATION**

(71) Applicant: Koninklijke Philips N.V., 5656 AG Eindhoven (NL)
(72) Inventor: ELENBAAS, Thijs, Eindhoven (NL); KOSSEN, Rebecca, Eindhoven (NL); NETO FONSECA, Lucia Teresa, 5656AG Eindhoven (NL); SPIJKERBOER, Koen, Eindhoven (NL)
(74) Representative: Philips Intellectual Property & Standards

(57) **Abstract**

The present invention relates to supporting subjects in medical interventions or imaging procedures. In order to provide a slim but rigid design of an interventional table, a subject support (10) with misalignment compensation for medical interventions is provided that comprises a support structure (12), an upper subject lying area (14), a misalignment measurement arrangement (16) and an adjustment arrangement (18). The support structure carries the upper subject lying area. The support structure is configured to be mounted to a support base. When a subject is arranged on the upper subject lying area, the misalignment measurement arrangement is configured to detect a degree of deformation of the support structure resulting in misalignment of the upper subject lying area. The support structure comprises at least partly smart material (20) that provides a determined degree of change of shaping upon receiving a trigger input. The adjustment arrangement is configured to provide the trigger input based on the detected degree of deformation. The determined degree of change of shaping at least partly compensates the degree of deformation.

## Description

### FIELD OF THE INVENTION

The present invention relates to a subject support with misalignment compensation for medical interventions, to a subject support arrangement for medical interventions, to a medical imaging system and to a method for misalignment compensation for medical interventions.

### BACKGROUND OF THE INVENTION

For medical interventions, height adjustable subject support, known as tables, are provided for supporting a patient during the intervention, for example in a laying manner. These tables are also referred to as patient tables. Another term is subject table or object table, also referred to as object support. A further term that is used is interventional table. In order to avoid deformation of the support when a subject, e.g. a patient, lays on such a patient table, a strong and rigid constructional design is chosen. Besides different weights of patients, also adjustability of the patient tables has to be considered in their structural setup. As an example, the patient table should be adjustable in height. In some examples, the patient table should also be adjustable along its longitudinal axis, i.e. lengthwise, or in its inclination, i.e. in the table's angle towards the horizontal plane. On top of that, for compatibility with X-Ray imaging, at least the part of the table extending over a base portion should also be X-ray transparent. However, it has been shown that the table itself, as well as its base, may be rather bulky to ensure a high stiffness and thus a minimum range of tolerances.

### SUMMARY OF THE INVENTION

There may thus be a need for a slim but rigid design of a subject support.

The object of the present invention is solved by the subject-matter of the independent claims; further embodiments are incorporated in the dependent claims. It should be noted that the following described aspects of the invention apply also for the subject support with misalignment compensation for medical interventions, for the subject support arrangement for medical interventions, for the medical imaging system and for the method for misalignment compensation for medical interventions.

According to the present invention, a subject support with misalignment compensation for medical interventions is provided. The subject support comprises a support structure, an upper subject lying area, a misalignment measurement arrangement and an adjustment arrangement. The support structure carries the upper subject lying area. The support structure is configured to be mounted to a support base. The misalignment measurement arrangement is configured to detect a degree of deformation of the support structure, when a subject is arranged on the upper subject lying area, resulting in misalignment of the upper subject lying area. The support structure comprises at least partly smart material that provides a determined degree of change of shaping upon receiving a trigger input. The adjustment arrangement is configured to provide the trigger input based on the detected degree of deformation. The determined degree of change of shaping at least partly compensates the degree of deformation.

As an effect, the problem of e.g. bending in a CT system is addressed. This will not only ensure that the support, i.e. the table, will always fit the bore of the CT gantry, but also - when local compensation is accurate enough - that both modalities remain registered in case two different imaging modalities are used.

According to an example, the smart material is integrated in the support structure such that the smart material, upon being exposed to the trigger signal, provides an upward force.

The upwards force is counteracting against the bending directed downwards due to gravity.

In an option, provided on addition or alternatively, the smart material is a combination of multiple materials with different properties and the behavior is determined by the interaction of differently chosen geometries of the multiple materials.

According to an example, the smart material is implemented within at least one support structure part such that changes in the shaping of the smart material result in changes in length of the respective support structure part.

The controllable length extension due to the smart material thus provides a sort of gear or lever mechanism that allows small changes in the shaping by the smart material leading to larger changes in the structure.

According to an example, at least a part of the support structure comprising the smart material is made by an additive manufacturing procedure.

This allows a targeted fabrication of components that can be combined with the surrounding structure in an integrated manner.

According to an example, the support structure is a 3D-printed structure.

According to an example, the misalignment measurement arrangement comprises at least one sensor that detects the degree of deformation and a data processor that is configured to receive the detected degree of deformation and to compute the trigger input provided to the smart material. The adjustment arrangement comprises a generator configured to generate the trigger input to be applied to the smart material.

According to an example, the generator generates an electric trigger input in form of a change of an applied voltage applied to the smart material.

According to an example, the trigger input is a change of at least one of the group of i) temperature affecting the smart material, ii) light affecting the smart material, iii) pressure affecting the smart material, iv) a pH of an environment affecting the smart material and v) chemical compounds affecting the smart material.

According to the present invention, also a subject support arrangement for medical interventions is provided. The subject support arrangement comprises a support base and a subject support according to one of the preceding examples. The support structure is mounted to the support base.

According to the present invention, also a medical imaging system is provided. The system comprises at least one imaging device and a subject support arrangement according to the preceding example. The subject support arrangement is configured for arranging a subject on the subject support arrangement during imaging by the at least one imaging device.

According to the present invention, also a method for misalignment compensation for medical interventions is provided. The method comprises the following steps:
- Arranging a subject on an upper subject lying area carried by a support structure of a subject support mounted to a support base; the support structure comprises at least partly smart material that provides a determined degree of change of shaping upon receiving a trigger input;
- Detecting, by a misalignment measurement arrangement, a degree of deformation of the support structure due to the subject being arranged on the upper subject lying area resulting in misalignment of the upper subject lying area;
- Providing, by an adjustment arrangement, the trigger input based on the detected degree of deformation; and
- Supplying the trigger input to the smart material resulting in a determined degree of change of shaping that at least partly compensates the degree of deformation.

As an advantageous effect, e.g. in an AngioCT system, bending of the subject support will be minimized, if not completely compensated, to avoid or decrease errors in the volume registration between the angiography system and the CT system. It is also avoided that the table tip is bended so far downwards that it would hit the bore of the CT scanner, i.e. the edges forming the center opening of a CT gantry. The solution outlined above mitigates this bending by use of the so-called 4D printing. The mix of different materials applied by 3D printing allows targeted deformation, which adds to the fourth dimension. The use of multi-material additive manufacturing creates structures that can be made to (de-)form under external stimuli and thereby provide a counter force against the bending force in a table. This creates a table that compensates for the local bending effect while still being thin and radio transparent, improving angiography CT compatibility and reducing any registration issues in multimodal applications.

As an effect, bending is reduced while actually avoiding a more structurally rigid table, which is also avoiding a higher or more X-ray dense table. Hence, both undesirable properties are avoided.

By addressing the bending with active counter measures, errors in image registration between e.g. a spectral CT and a further imaging are avoided, especially when the table is moved through the CT, since this extends the table further from its base.

According to an aspect, a support structure is provided with smart material that can be activated by a generated signal in order to provide a targeted change of shaping that addresses a detected deformation when a subject is resting on the support. The smart material compensates for the bending or other deformation such that the resulting support is aligned again.

According to an aspect, bending is addressed within the structure, instead of further adjustments e.g. of a movement system of the support structure.

In an option, the smart material activation is also used for fine adjustment of the subject support.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a subject support with misalignment compensation for medical interventions.
Fig. 2 shows an illustration of a concept of misalignment compensation.
Fig. 3 shows an example of a subject support arrangement in the context of an example of a medical imaging system.
Fig. 4 shows basic steps of an example of a method for misalignment compensation for medical interventions.

### DETAILED DESCRIPTION OF EMBODIMENTS

Certain embodiments will now be described in greater details with reference to the accompanying drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

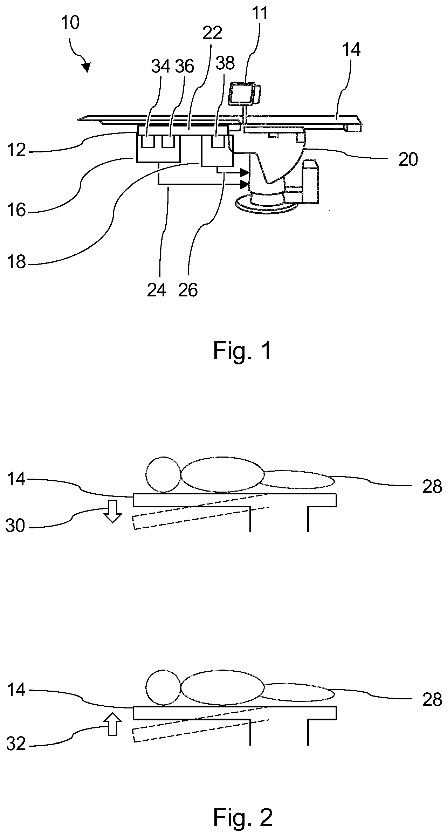

Fig. 1 schematically shows an example of a subject support 10 with misalignment compensation for medical interventions. The subject support 10 comprises a support structure 12, an upper subject lying area 14, a misalignment measurement arrangement 16 and an adjustment arrangement 18. The support structure 12 carries the upper subject lying area 14. The support structure 12 is configured to be mounted to a support base 20. When a subject, such as a patient (not shown in Fig. 1), is arranged on the upper subject lying area 14, the misalignment measurement 16 arrangement is configured to detect a degree of deformation of the support structure 12 resulting in misalignment of the upper subject lying area 14. The support structure 12 comprises at least partly smart material 22 that provides a determined degree of change of shaping upon receiving a trigger input. The adjustment arrangement 18 is configured to provide the trigger input based on the detected degree of deformation. The determined degree of change of shaping at least partly compensates the degree of deformation.

The expression "least partly smart material" means that a part of the support structure is made from smart material. However, the expression "least partly smart material" can also mean that a part of the material of a part or the complete support structure comprises smart material.

In Fig. 1, the misalignment measurement arrangement 16 and the adjustment arrangement 18 are schematically shown as separate boxes connected by communication connections 24 and 26. It is noted that the misalignment measurement arrangement 16 and the adjustment arrangement 18 can also be provided in a more integrated manner or also in a more separated manner.

A smaller monitor or control interface 11 can provided next to the subject support 10.

Fig. 2 shows an illustration of the concept of misalignment compensation. As indicated in an upper part of Fig. 2, a subject 28 arranged on the upper subject lying area 14 urges on the construction caused by the subject's weight, which results in a bending of the support structure 12 carrying the upper subject lying area 14, as indicated with a first arrow 30 pointing downwards. The smart material 22 is activated and the bending is compensated for, as indicated in a lower part of Fig. 2 with a second arrow 32 pointing upwards.

The term "subject support" relates to holding a patient in place during the medical intervention. The subject support 10 can also be referred to as subject table or patient table or operation table. The medical intervention can be an examination procedure, an interventional procedure for diagnosis or treatment. The medical intervention can also be an imaging procedure.

The term "support structure" relates to the part of the subject support 10 that transfers load from the subject resting on the upper lying area 14 to a base, e.g. standing on a floor construction. The support structure 12 can be provided as a frame or grid or other type of lightweight but rigid construction, for example made from non-metal material such as fiber-reinforced plastics. The support structure 12 can also be referred to as frame or body structure.

The term "upper subject lying area" relates to the surface provided to the subject for resting on during imaging or other medical procedure.

The term "misalignment measurement arrangement" relates to a setup provided for measuring the misalignment. The misalignment measurement arrangement 16 can also be referred to as misalignment detection device or misalignment sensor arrangement.

The term "adjustment arrangement" relates to setup provided for adjusting the misalignment such that it is less then without the setup. The adjustment arrangement 18 can also be referred to adjustment device or adjustment setup.

The term "change of deformation" relates to an unwanted change of the support when being subject to a load of a subject resting on the support. The deformation takes place, for example, within the structure, and comprises a bending of the support.

The term "change of shaping" relates to adapting the shape or form of the respective part or portion.

The change of shaping can also be referred to as the change of the shape or as change in the formation of the respective part or portion. The change in the formation is so-to-speak a matching active reaction upon detecting a deformation. The change in the formation, or change of formation, is the counterpart to the change in deformation. While the term "deformation" refers to a passive change in the shape leading to an unwanted misalignment, the term "formation" refers to an active change in the shape leading to the wanted compensation of the unwanted misalignment.

The term "trigger input" relates to a change of a parameter that is suitable to activate the change in the shaping of the smart material.

The term "smart material" relates to a material that is capable of achieving a second state or form when a certain input is applied. The input acts as a signal, and a change is thereby triggered. Hence the term "smart".

The misalignment can also be referred to as displacement.

In an example, the misalignment measurement arrangement 16 is configured to detect a misalignment of at least one parameter of the group of:
i) a degree of deformation of the support structure 12, and
ii) a resulting displacement of the upper subject lying area 14 in relation to the support base.

The term "smart materials" relates to materials that have properties that can change due to external stimuli or condition changes. The stimuli can be provided as trigger input.

In an example, the smart material is a combination of multiple materials with different properties under applied stimuli.

As an option, the smart material is integrated in the support structure 12 such that the smart material, upon being exposed to the trigger signal, provides an upward force.

The term "upward force" relates to a state of normal use, i.e. when a subject is resting on the support bending the structure at least partly in a downward direction. The force generated or provided by the smart material in the support structure 12 has a vector pointing upwards, i.e. in an opposite direction.

As an option, the smart material is implemented within at least one support structure part such that changes in the shaping of the smart material result in changes in length of the respective support structure part.

The term "support structure part" relates to a portion of the support structure 12. The part can be a separate component like a rod or beam, or can be a portion within a component. The support structure part can also be referred to as support structure portion or segment.

As an option, at least a part of the support structure 12 comprising the smart material is made by an additive manufacturing procedure. As an option, the smart material is a combination of multiple materials with different properties and the behavior is determined by the interaction of differently chosen geometries of the multiple materials.

The term "additive manufacturing" relates to a procedure in which the structure is continuously fabricated by adding material. An example of such additive manufacturing is 3D printing.

As an option, the support structure 12 is a 3D-printed structure.

The term "3D-printed structure" relates to the complete structure being manufactured by 3D printing.

As an option, the misalignment measurement arrangement 16 comprises at least one sensor 34 that detects the degree of deformation and a data processor 36 that is configured to receive the detected degree of deformation and to compute the trigger input provided to the smart material. The adjustment arrangement 18 comprises a generator 38 configured to generate the trigger input to be applied to the smart material.

The term "sensor" relates to a device for detecting the degree of deformation. This can be provided e.g. by an optical sensor, an electromagnetic sensor or by other means to detect the deformation.

The term "data processor" relates to a unit capable of processing the sensor data in order to generate or output a stimulus as input for the smart material. The data processor 36 (for the smart material) can be a separate minor data processor or can be integrated in a processor provided for other purposes as well, such as for controlling a movement of the support in vertical and horizontal direction as well as inclination adjustment. Basically, the data processor 36 receives the sensor output and compares this with a threshold in order to generate a trigger signal when exceeding the threshold.

The term "generator" relates to providing a signal acting as the trigger input. The generator 38 can be part of the data processor 36 or can be a separate component.

Thus, for the subject support, new hardware is provided in form of the structure with the smart material and software in form of the measuring and trigger signal generation. In an option, a feedback loop of measuring deformation & applying the trigger signal is provided. The trigger signal can also be referred to as stimuli.

In an example, at least the part of the support structure 12 that comprises the smart material is a 3D-printed structure.

The combination of 3D printing and smart, deformable, materials can also be referred to as 4D printing.

Combining such smart materials with additive manufacturing results in a 3D-printed object with tailored properties that change with the proper stimuli.

The integration of 4D printed structures into the Angio CT table, addresses the table deformation.

Using 4D printing deformable materials allows the creation of a table with an adaptive counter force for a range of table loads and table positions. By designing a specific structure, the material will provide the required upward force to counter the downward force of the weight on every position along the table, while not requiring a thicker or much denser table. The table can also be referred to as 4D table.

In an example, an algorithm is provided to determine appropriate stimulus to counteract table deformation.

As an option, the generator 38 generates an electric trigger input in form of a change of an applied voltage applied to the smart material.

As another option, the trigger input is a change of at least one of the group of: temperature affecting the smart material, light affecting the smart material,
pressure affecting the smart material, a pH of an environment affecting the smart material and chemical compounds affecting the smart material.

In a further option, a combination of different stimuli is provided.

As an option, a feedback loop is provided by the misalignment measurement arrangement 16 to determine an achieved misalignment with the trigger input and to determine a remaining misalignment. The adjustment arrangement 18 is configured to provide a further trigger input to achieve an increased degree of compensation.

In an example, the adjustment arrangement 18 is configured to provide an approximation trigger input which generates less change of shaping of the smart material then the trigger input. The feedback loop by the misalignment measurement arrangement 16 is provided to determine the achieved misalignment measurement arrangement 16 with the approximation trigger input and to determine the remaining misalignment. The adjustment arrangement 18 is configured to provide a further trigger input to achieve an increased degree of compensation.

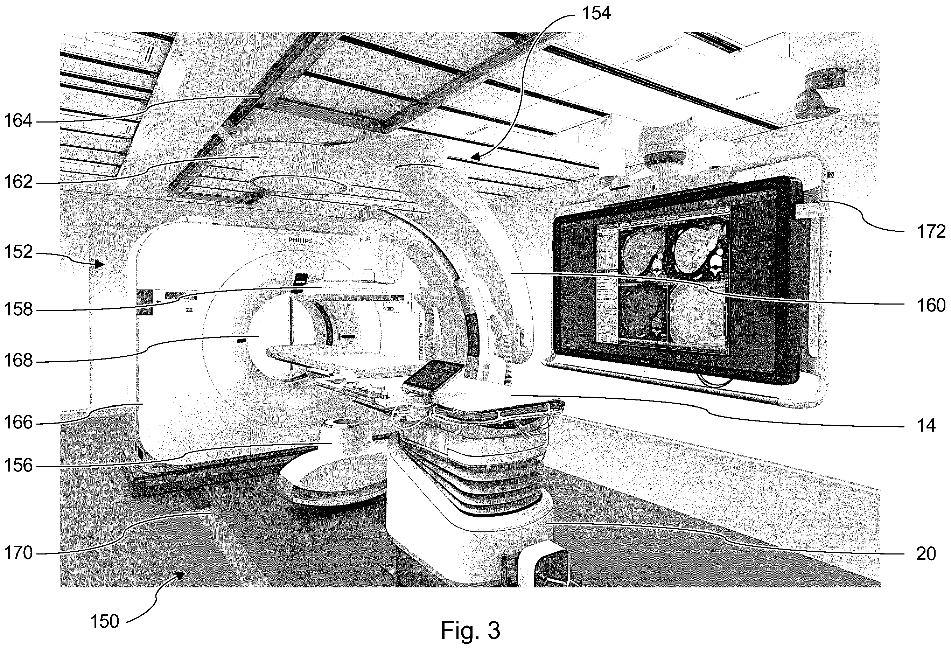

Fig. 3 shows an example of a subject support arrangement 100 in the context of an example of a medical imaging system 150.

The subject support arrangement 100 comprises a support base 102 and a subject support 10 according to one of the preceding examples. The support structure 10 is mounted to the support base 102.

The term "support base" relates to a stand mounted to or resting on a rigid building structure. The support base can be fixedly mounted or can be movable mounted on rails or can be a movable base, e.g. with a passive or active drive mechanism.

In an example, shown in Fig. 3 as an option, the support structure 12 is movably mounted to the support base 102. The misalignment measurement arrangement 16 is configured to detect a misalignment after moving the support base 102 with the subject being arranged on the upper subject lying area 14.

In an example, the tabletop can be moved in horizontal direction to change the amount of extension or extraction of the table, i.e. a degree of a cantilevering of a part of the table.

As indicated above, Fig. 3 also shows an example of the medical imaging system 150 comprising at least one imaging device 152 and the subject support arrangement 100 according to the examples above. The subject support arrangement 100 is configured for arranging a subject on the subject support arrangement 100 during imaging by the at least one imaging device 152.

In an option (not shown), the subject support table is fixedly mounted.

In another option (as shown), the subject table is movably mounted. For example, the subject support can be adjusted in height. In another example, provided alternatively or in addition, the subject support can be adjusted in its inclination to the horizontal, e.g. as being pivotable around a longitudinal horizontal axis and/or a transverse horizontal axis. In a further example, provided alternatively or in addition, the subject support can be adjusted in its position along a horizontal plane, e.g. being slidable in a longitudinal horizontal and/or a transverse direction. The movement options can be provided by a base. Also, a rail system in the floor can be provided.

The medical imaging system 150 can also be referred to as medical imaging arrangement, and the imaging device(s) can also be referred to as imaging system(s).

As an option, it is provided that the at least one imaging device 152 comprises at least one of the group of: a radiography imaging device, a magnetic resonance tomography device, a nuclear imaging device and an ultrasound imaging device.

As an example, the radiographic imaging device is provided as an angiography X-ray imaging device using a C-arm, or as CT imaging device or as conebeam CT imaging device. As another example, the imaging device applies magnetic resonance imaging (MRI). As a further example, nuclear imaging is provided as SPECT or PET.

As an option, Fig. 3 shows that two different imaging devices are provided. The subject support 10 of the subject support arrangement 100 is configured to be moved between imaging by the two imaging devices. A misalignment of the support structure 12 of the subject support 10 is compensated by activating the smart material.

As an example, Fig. 3 shows a C-arm X-ray imaging system 154 with an X-ray source 156 and an X-ray detector 158 mounted to ends of a movable C-arm 160. The C-arm may be suspended from a ceiling with a support structure 162. A rail system 164 may be arranged in an upper area for additional movement possibilities.

As a further example, Fig. 3 shows a CT gantry 166 with an aperture 168 for receiving a subject (not shown) arranged on a subject support. In an option, the CT gantry 166 is movable along floor mounted rails 170.

In addition, a display arrangement or monitor 172 is arranged in the vicinity.

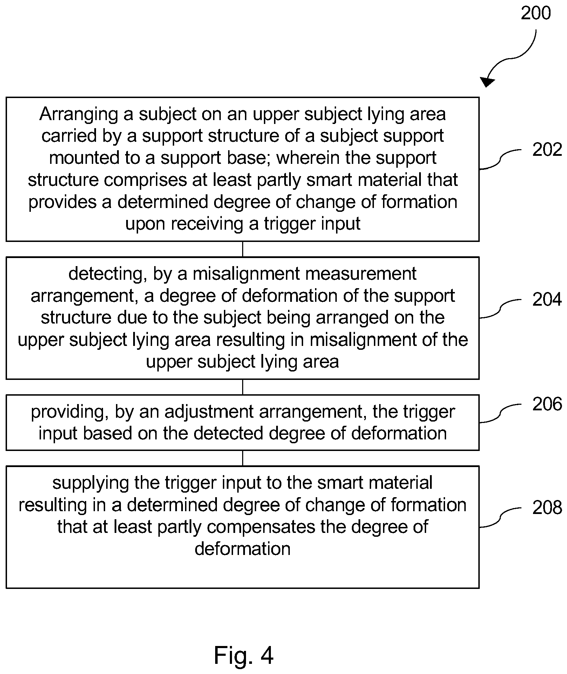

Fig. 4 shows basic steps of an example of a method 200 for misalignment compensation for medical interventions, the method comprising the following steps:
- In a first step 202, a subject is arranged on an upper subject lying area carried by a support structure of a subject support mounted to a support base; the support structure comprises at least partly smart material that provides a determined degree of change of shaping upon receiving a trigger input.
- In a second step 204, a degree of deformation of the support structure due to the subject being arranged on the upper subject lying area resulting in misalignment of the upper subject lying area is detected by a misalignment measurement arrangement.
- In a third step 206, the trigger input based on the detected degree of deformation is provided by an adjustment arrangement.
- In a fourth step 208, the trigger input is supplied to the smart material resulting in a determined degree of change of shaping that at least partly compensates the degree of deformation.

A field of use are e.g. angiography computer tomography imaging systems (AngioCT systems) where it is important that the table does not bend excessively, e.g. hitting the CT bore, and where it is important that the CT image data and the X-ray image data remain registered. In an example, the angiography imaging is provided by a movably mounted C-arm structure and the CT imaging is provided by a fixedly mounted CT scanner with a gantry. A further field of use are applications where table compensation in a lightweight table is required.

The term "subject" may also be referred to as individual. The "subject" may further also be referred to as patient, although it is noted that this term does not indicate whether any illness or disease is actually present with the subject.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A subject support (10) with misalignment compensation for medical interventions, the subject support comprising:
- a support structure (12);
- an upper subject lying area (14);
- a misalignment measurement arrangement (16); and
- an adjustment arrangement (18);
wherein the support structure carries the upper subject lying area; and wherein the support structure is configured to be mounted to a support base;
wherein, when a subject is arranged on the upper subject lying area, the misalignment measurement arrangement is configured to detect a degree of deformation of the support structure resulting in misalignment of the upper subject lying area;
wherein the support structure comprises at least partly smart material (20) that provides a determined degree of change of shaping upon receiving a trigger input;
wherein the adjustment arrangement is configured to provide the trigger input based on the detected degree of deformation; and
wherein the determined degree of change of shaping at least partly compensates the degree of deformation.

2. Subject support according to claim 1, wherein the smart material is integrated in the support structure such that the smart material, upon being exposed to the trigger signal, provides an upward force.

3. Subject support according to claim 1 or 2, wherein the smart material is implemented within at least one support structure part such that changes in the shaping of the smart material result in changes in length of the respective support structure part.

4. Subject support according to claim 1, 2 or 3, wherein at least a part of the support structure comprising the smart material is made by an additive manufacturing procedure;
wherein, preferably, the smart material is a combination of multiple materials with different properties and the behavior is determined by the interaction of differently chosen geometries of the multiple materials.

5. Subject support according to claim 4, wherein the support structure is a 3D-printed structure.

6. Subject support according to one of the preceding claims, wherein the misalignment measurement arrangement comprises:
- at least one sensor (34) that detects the degree of deformation; and
- a data processor (36) that is configured to receive the detected degree of deformation and to compute the trigger input provided to the smart material; and
wherein the adjustment arrangement comprises:
- a generator (38) configured to generate the trigger input to be applied to the smart material.

7. Subject support according to one of the preceding claims, wherein the generator generates an electric trigger input in form of a change of an applied voltage applied to the smart material.

8. Subject support according to one of the preceding claims, wherein the trigger input is a change of at least one of the group of:
i) temperature affecting the smart material;
ii) light affecting the smart material;
iii) pressure affecting the smart material;
iv) a pH of an environment affecting the smart material; and
v) chemical compounds affecting the smart material.

9. Subject support according to one of the preceding claims, wherein a feedback loop is provided by the misalignment measurement arrangement to determine an achieved misalignment arrangement with the trigger input and to determine a remaining misalignment, and
wherein the adjustment arrangement is configured to provide a further trigger input to achieve an increased degree of compensation.

10. A subject support arrangement (100) for medical interventions, the subject support arrangement comprising:
- a support base (102); and
- a subject support (10) according to one of the preceding claims;
wherein the support structure is mounted to the support base.

11. Support arrangement according to claim 10, wherein the support structure is movably mounted to the support base; and
wherein the misalignment measurement arrangement is configured to detect a misalignment after moving the support base with the subject being arranged on the upper subject lying area.

12. A medical imaging system (150), comprising:
- at least one imaging device (152); and
- a subject support arrangement (100) according to claim 10 or 11;
wherein the subject support arrangement is configured for arranging a subject on the subject support arrangement during imaging by the at least one imaging device.

13. System according to claim 12, wherein the at least one imaging device comprises at least one of the group of:
- radiography imaging device;
- magnetic resonance tomography device;
- nuclear imaging device; and
- ultrasound imaging device.

14. System according to claim 12 or 13, wherein two different imaging devices are provided;
wherein the subject support of the subject support arrangement is configured to be moved between imaging by the two imaging devices; and
wherein a misalignment of the support structure of the subject support is compensated by activating the smart material.

15. A method (200) for misalignment compensation for medical interventions, the method comprising the following steps:
- arranging (202) a subject on an upper subject lying area carried by a support structure of a subject support mounted to a support base; wherein the support structure comprises at least partly smart material that provides a determined degree of change of shaping upon receiving a trigger input;
- detecting (204), by a misalignment measurement arrangement, a degree of deformation of the support structure due to the subject being arranged on the upper subject lying area resulting in misalignment of the upper subject lying area;
- providing (206), by an adjustment arrangement, the trigger input based on the detected degree of deformation; and
- supplying (208) the trigger input to the smart material resulting in a determined degree of change of shaping that at least partly compensates the degree of deformation.
